# EUROPÄISCHE PATENTSCHRIFT

(11) **EP 0 761 639 B1**
(45) Veröffentlichungstag und Bekanntmachung des Hinweises auf die Patenterteilung: **26.08.1998**
(21) Anmeldenummer: 96110260.5
(22) Anmeldetag: 26.06.1996
(51) Int. Cl.: C07C 67/39, C07C 67/48, C07C 69/82

(54) **Verfahren zur Aufarbeitung von Rückständen aus der Rohester-Destillation bei der Herstellung von Dimethylterephthalat (DMT)**
Process for the treatment of residues from the crude ester distillation from the preparation of dimethyl terephthalate (DMT)
Procédé pour le traitement de résidus de la distillation de l'ester brut lors de la préparation de téréphtalate de diméthyle (DMT)

(30) Priorität: 23.08.1995 DE 19530970
(43) Veröffentlichungstag der Anmeldung: 12.03.1997
(73) Patentinhaber: HÜLS AKTIENGESELLSCHAFT, 45764 Marl (DE)
(72) Erfinder: Auschner, Reinhard, 53844 Troisdorf (DE); Thiel, Ralf, 53859 Niederkassel (DE)

(56) Entgegenhaltungen:
- WO-A-90/09367
- DE-A- 2 244 662
- DE-B- 1 142 858

## Beschreibung

Die Erfindung betrifft ein Verfahren zur Aufarbeitung einer Rückstandsfraktion durch Methanolyse, wobei die Rückstandsfraktion aus der Rohester-Destillation des DMT-Herstellprozesses stammt.

Dimethylterephthalat (DMT) wird großtechnisch in zahlreichen Anlagen weltweit hergestellt. DMT ist eine wichtige Ausgangsverbindung für die Herstellung von Polyestern. Die Anwendungsgebiete der Polyester für Fasern sowie Folien, u. a. für fotographische Filme und Magnetbänder oder Flaschen aus Polyethylenterephthalat, um nur einige zu nennen, sind lange bekannt.

Das heutige Witten-DMT-Verfahren beinhaltet (vgl. EP-PS 0 464 046, DE-OS 40 26 733) im wesentlichen die Verfahrensschritte
- Oxidation von para-Xylol (p-X) und para-Toluylsäure-methylester (p-TE) mit nachgeschalteter Abgasreinigung
- Veresterung der Reaktionsprodukte aus der Oxidation mit Methanol
- Trennung des entstandenen sogenannten Rohesters in
   a) eine Fraktion, die in die Oxidation zurückgeführt wird,
   b) eine Roh-DMT-Fraktion mit mehr als 99 Gew.-% DMT und
   c) eine schwersiedende Rückstandsfraktion einschließlich deren Aufarbeitung,
- Reinigung der Roh-DMT-Fraktion, beispielsweise durch Waschen, Umkristallisieren sowie Reindestillation.

Es ist auch möglich, aus DMT-reichen Fraktionen durch eine gezielte Hydrolyse Terephthalsäure herzustellen.

Die Oxidation eines Gemisches aus para-Xylol (p-X) und para-Toluylsäuremethylester (p-TE oder pT-Ester) wird im allgemeinen mit Luftsauerstoff in Gegenwart eines Schwermetallkatalysators (DE-PS 20 10 137) bei einer Temperatur von etwa 140 bis 180 °C und einem Druck von etwa 4 bis 8 bar abs. in flüssiger Phase durchgeführt. Aus der Oxidationsstufe resultiert ein Reaktionsgemisch, das überwiegend Monomethylterephthalat (MMT), p-Toluylsäure (p-TA) und Terephthalsäure (TA), gelöst bzw. suspendiert in p-TE, enthält und mit Methanol bei einer Temperatur von etwa 250 bis 280 °C und einem Druck von 20 bis 25 bar abs. verestert wird. Der erhaltene Rohester wird destillativ in eine p-TE-Fraktion, eine Roh-DMT-Fraktion und eine hochsiedende, Katalysator-haltige Rückstands-Fraktion aufgetrennt. Die p-TE-Fraktion wird in die Oxidation zurückgeführt und die Roh-DMT-Fraktion über nachfolgende Reinigungsstufen in die gewünschte Produktqualität überführt.

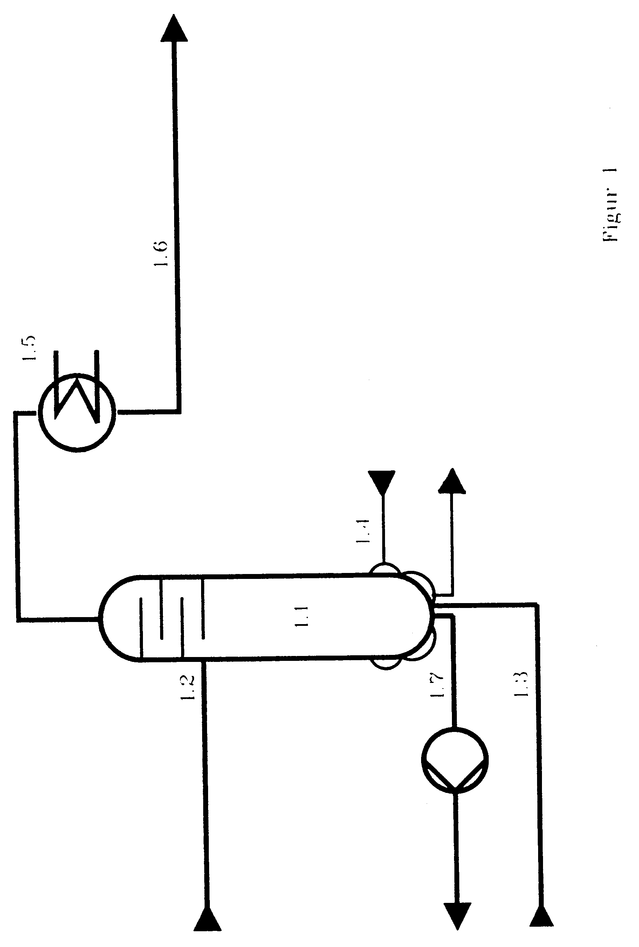

Die aus der Rohester-Destillation stammende Rückstandsfraktion wird im allgemeinen in einer Methanolyse nachbehandelt. Figur 1 zeigt das Fließschema einer einstufigen Methanolyse. In die Reaktionsdestillationskolonne (1.1) werden kontinuierlich besagte Rückstände (1.2) und überhitzter Methanoldampf (1.3) drucklos im Gegenstrom eingeleitet. Der Sumpf der Kolonne wird zusätzlich über ein Wärmeträgeröl beheizt (1.4). Die Methanolyse wird bei einer Temperatur von 265 bis 280 °C betrieben. Dabei wird ein Teil der Rückstände in Stoffe überführt, die für den Prozeß wiederverwertbar sind. In der Rückstandsfraktion vorhandene Säuren werden in der Methanolyse nachverestert, ein Teil der hochsiedenden organischen Verbindungen gespalten und bereits vorhandene organische Wertprodukte von unerwünschten, nicht mehr verwertbaren organischen Verbindungen getrennt. Die so erhaltenen Wert- bzw. Nutzstoffe gelangen zusammen mit überschüssigem Methanol über den Kolonnenkopf in den Dephlegmator (1.5) und werden anschließend in den Prozeß, d. h. in die Oxidation, zurückgeführt (1.6). Den bei der Methanolyse anfallenden Sumpfrückstand führt man im allgemeinen der Katalysatorrückgewinnung zu (1.7). Die Praxis zeigt, daß es in der Methanolyse zu Verkokungen und Verstopfungen in der Reaktionskolonne kommt - die Folge sind häufigere und außerplanmäßige Abstellungen der Anlageneinheit.

Aus der EP-PS 0 464 046 ist eine zweistufige Methanolyse für die Aufarbeitung des Rückstandes aus der Rohester-Destillation bekannt. Die erste Methanolyse-Stufe besteht hier im wesentlichen aus einem Reaktor mit vorgeschaltetem Wärmetauscher und Umwälzsystem, dem Reaktor ist kopfseitig eine Destillationskolonne nachgeschaltet. Die zweite Methanolyse-Stufe beinhaltet im Gegensatz zur ersten eine Reaktionsdestillationskolonne. In beiden Stufen wird zusätzliches dampfförmiges Methanol bereits vor Wärmetauscher, d. h. also vor Eintritt in die jeweiligen Methanolysereaktoren, in den jeweiligen Kreislaufstrom der Rückstände eingespeist. Die Betriebstemperaturen werden für beide Methanolyse-Stufen mit Temperaturen von mehr als 265 °C angegeben, das betrifft insbesondere die zweite Methanolyse-Stufe. Jede Methanolyse-Stufe wird ferner in Kreisfahrweise betrieben und nur Teilströme ersetzt, wobei natürlich die Durchsatzmengen hinsichtlich der aufzuarbeitenden Rückstandsfraktion vergleichsweise gering sind. Zusätzlich erfordert die zweistufige Methanolyse ein hohes Investment und für das Betreiben der Anlage hohe Instandhaltungskosten.

Der Erfindung lag daher die Aufgabe zugrunde, ein Verfahren bereitzustellen, das es ermöglicht, die bei der Rohester-Destillation anfallende Rückstandsfraktion möglichst wirtschaftlich aufzuarbeiten und insbesondere Verkokungen in den Reaktoren und Leitungssytemen der Methanolyse zu mindern.

Es wurde nun überraschenderweise gefunden, daß bei einer Fahrweise der Methanolyse, die durch die Zugabe von flüssigem und/oder dampfförmigem Methanol zum Rohester-Destillat, einer Gemischtemperatur vor Eintritt in den Methanolysereaktor zwischen 230 und 265 °C und einer Temperatur im Reaktorsumpf zwischen 230 und 265 °C, gekennzeichnet ist, Verkokungen weitgehend reduziert werden konnten. Insbesondere wird im vorliegenden Verfahren der Reaktorsumpf keiner den Durchsatz mindernden Kreisfahrweise unterzogen, wodurch die Kapazität der Methanolyse-Stufe deutlich gesteigert werden konnte.

Gegenstand der vorliegenden Erfindung ist daher ein Verfahren zur Aufarbeitung einer Rückstandsfraktion durch Methanolyse, wobei die Rückstandsfraktion aus der Rohester-Destillation des DMT-Herstellprozesses stammt, das dadurch gekennzeichnet ist, daß man der Rückstandsfraktion flüssiges und/oder dampfförmiges Methanol zuführt, das Gemisch vor Eintritt in den Methanolysereaktor eine Temperatur zwischen 230 und 265 °C, vorzugsweise von 245 bis 255 °C, besitzt und die Temperatur im Reaktorsumpf ebenfalls zwischen 230 und 265 °C, vorzugsweise bei 245 bis 255 °C, liegt. Geeigneterweise betreibt man die Methanolyse bei einem Druck von 1 bis 40 bar abs., vorzugsweise bei einem Druck von 1 bis 3 bar abs..

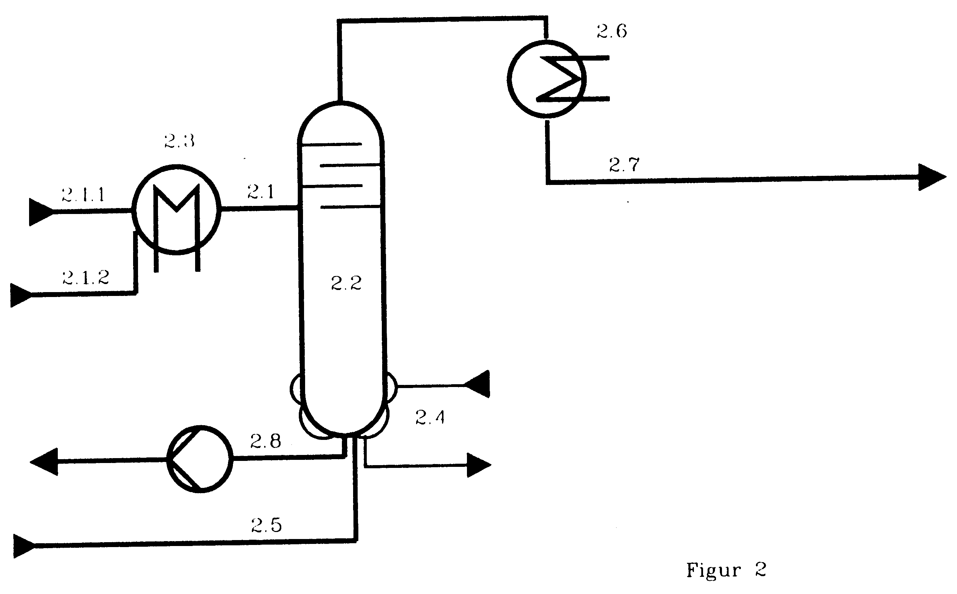

Figur 2 zeigt ein Fließschema einer bevorzugten Ausführungsform des erfindungsgemäßen Verfahrens, wobei man die Methanolyse insbesondere einstufig betreibt und die Rückstandsfraktion aus der Rohester-Destillation (2.1.1) vorzugsweise in einer Reaktionsdestillationskolonne (2.2) aufarbeitet. Geeigneterweise führt man dabei die mit Methanol (2.1.2) versetzte Rückstandsfraktion vor Eintritt in den Methanolysereaktor über einen Wärmetauscher (2.3). Der Wärmetauscher kann elektrisch und/oder mit hochgespanntem Dampf und/oder mit einem vorgeheizten Wärmeträgeröl, beispielsweise MARLOTHERM®, betrieben werden. Auch der Reaktorsumpf (2.4) kann im erfindungsgemäßen Verfahren elektrisch und/oder mit hochgespanntem Dampf und/oder mit einem vorgeheizten Wärmeträgeröl beheizt werden. Geeigneterweise wird die, wie zuvor beschrieben, mit Methanol vorbehandelte Rückstandsfraktion im Methanolysereaktor unter weiterer Zufuhr von Methanol (2.5) umgesetzt, wobei auch hier das Methanol flüssig und/oder dampfförmig zugeführt werden kann. Die Umsetzung wird nach dem erfindungsgemäßen Verfahren unter besonders schonenden und unter vergleichsweise milden Bedingungen durchgeführt. Geeigneterweise werden die dabei erhältlichen Nutzprodukte zunächst über einen Dephlegmator (2.6) und dann in den Prozeß, d. h. in die Oxidations-Stufe, zurückgeführt (2.7). Der Anteil an Nutzprodukt im Sumpf der Reaktionskolonne konnte im erfindungsgemäßen Verfahren auf rund 3,5 % gegenüber etwa 8,5 % nach dem Stande der Technik reduziert werden. Beim erfindungsgemäßen Verfahren kann man einen Teil des Reaktorsumpfes ausschleusen und einem Verfahren zur Katalysatorrückgewinnung zuführen (2.8), wobei der Füllgrad des Reaktorsumpfes weitgehend konstant zu halten ist, was sich beispielsweise auch über die Zufuhrmenge des Rückstandes aus der Rohester-Destillation regeln läßt. Die Entnahme von Teilmengen aus dem Reaktorsumpf kann kontinuierlich oder diskontinuierlich erfolgen. Die Katalysatorrückgewinnung erfolgt beispielsweise durch Extraktion (vgl. EP-PS 0 053 241).

Durch die schonende Fahrweise im erfindungsgemäßen Verfahren konnte die Standzeit der Methanolyse verlängert werden, was einen weiteren wirtschaftlichen Vorteil darstellt. Besonders überraschend war, daß durch das erfindungsgemäße Verfahren sogar ohne Kreisfahrweise hinsichtlich des Reaktorsumpfes die Ausbeute über den gesamten DMT-Prozeß um rund 0,5 % gesteigert werden konnte - das ist eine zusätzliche Verbesserung der Wirtschaftlichkeit des Prozesses.

### Legende zu Figur 1:

Fließschema einer Methanolyse zur Aufarbeitung eines Rückstandes aus der Rohester-Destillation bei der Herstellung von DMT
- 1.1: Reaktionsdestillationskolonne
- 1.2: Zuführung des Rückstandes aus der Rohester-Destillation
- 1.3: Zuführung von dampfförmigem Methanol
- 1.4: Beheizung des Sumpfes der Reaktionskolonne
- 1.5: Dephlegmator
- 1.6: Zurückführung der Nutzprodukte in den Prozeß
- 1.7: Teilstrom des Sumpfrückstandes zur Katalysatorrückgewinnung

### Legende zu Figur 2:

Fließschema einer erfindungsgemäßen Methanolyse zur Aufarbeitung eines Rückstandes aus der Rohester-Destillation bei der Herstellung von DMT
- 2.1: Zuführung des Rückstandes (2.1.1) aus der Rohester-Destillation, dem Methanol (2.1.2) zugesetzt ist
- 2.2: Reaktionsdestillationskolonne
- 2.3: Wärmetauscher
- 2.4: Beheizung des Sumpfes der Reaktionskolonne
- 2.5: Zuführung von Methanol
- 2.6: Dephlegmator
- 2.7: Zurückführung der Nutzprodukte in den Prozeß
- 2.8: Teilstrom des Sumpfrückstandes zur Katalysatorrückgewinnung

## Patentansprüche

1. Verfahren zur Aufarbeitung einer Rückstandsfraktion durch Methanolyse, wobei die Rückstandsfraktion aus der Rohester-Destillation des DMT-Herstellprozesses stammt,
dadurch gekennzeichnet,
daß man der Rückstandsfraktion flüssiges und/oder dampfförmiges Methanol zuführt, das Gemisch vor Eintritt in den Methanolysereaktor eine Temperatur zwischen 230 und 265 °C besitzt und die Temperatur im Reaktorsumpf ebenfalls zwischen 230 und 265 °C liegt.

2. Verfahren gemäß Anspruch 1,
dadurch gekennzeichnet,
daß das Gemisch vor Eintritt in den Methanolysereaktor eine Temperatur von 245 bis 255 °C besitzt.

3. Verfahren nach Anspruch 1 oder 2,
dadurch gekennzeichnet,
daß die Temperatur im Reaktorsumpf 245 bis 255 °C beträgt.

4. Verfahren gemäß einem der vorhergehenden Ansprüche,
dadurch gekennzeichnet,
daß man die Methanolyse bei einem Druck von 1 bis 40 bar abs. betreibt.

5. Verfahren gemäß Anspruch 4,
dadurch gekennzeichnet,
daß man die Methanolyse bei einem Druck von 1 bis 3 bar abs. betreibt.

6. Verfahren gemäß einem der vorhergehenden Ansprüche,
dadurch gekennzeichnet,
daß man die mit Methanol versetzte Rückstandsfraktion vor Eintritt in den Methanolysereaktor über einen Wärmetauscher führt.

7. Verfahren gemäß einem der vorhergehenden Ansprüche,
dadurch gekennzeichnet,
daß der Wärmetauscher elektrisch und/oder mit hochgespanntem Dampf und/oder mittels vorgeheiztem Wärmeträgeröl betrieben wird.

8. Verfahren gemäß einem der vorhergehenden Ansprüche,
dadurch gekennzeichnet,
daß der Reaktorsumpf elektrisch und/oder mit hochgespanntem Dampf und/oder mittels vorgeheiztem Wärmeträgerol beheizt wird.

9. Verfahren gemäß einem der vorhergehenden Ansprüche,
dadurch gekennzeichnet,
daß die mit Methanol vorbehandelte Rückstandsfraktion im Methanolysereaktor unter weiterer Zufuhr von Methanol umgesetzt wird.

10. Verfahren gemäß einem der vorhergehenden Ansprüche,
dadurch gekennzeichnet,
daß die Rückstandsfraktion in einer Reaktionsdestillationskolonne aufgearbeitet wird.

11. Verfahren gemäß einem der vorhergehenden Ansprüche,
dadurch gekennzeichnet,
daß man den Reaktorsumpf keiner Kreisfahrweise unterzieht.

12. Verfahren gemäß einem der vorhergehenden Ansprüche,
dadurch gekennzeichnet,
daß man einen Teil des Reaktorsumpfes ausschleust und einem Verfahren zur Katalysatorrückgewinnung zuführt.

13. Verfahren gemäß einem der vorhergehenden Ansprüche,
dadurch gekennzeichnet,
daß man die Methanolyse einstufig betreibt.

14. Verfahren gemäß einem der vorhergehenden Ansprüche,
dadurch gekennzeichnet,
daß dabei erhältliche Nutzstoffe in den Prozeß zurückgeführt werden.

## Claims

1. A process for working up a residue fraction by methanolysis, the residue fraction originating from the raw ester distillation of the DMT production process, characterized in that livid and/or gaseous methanol is fed into the residue fraction, the mixture before entry into the methanolysis reactor has a temperature of from 230 to 265°C and the temperature in the bottom of the reactor is likewise from 230 to 265°C.

2. A process according to claim 1, characterized in that the mixture before entry into the methanolysis reactor has a temperature of from 245 to 255°C.

3. A process according to claim 1 or 2, characterized in that the temperature in the bottom of the reactor is from 245 to 255°C.

4. A process according to any one of the preceding claims, characterized in that the methanolysis is carried out at a pressure of from 1 to 40 bar abs.

5. A process according to claim 4, characterized in that the methanolysis is carried out at a pressure of from 1 to 3 bar abs.

6. A process according to any one of the preceding claims, characterized in that the residue fraction admixed with methanol is conducted via a heat exchanger before entry into the methanolysis reactor.

7. A process according to any one of the preceding claims, characterized in that the heat exchanger is operated electrically and/or by means of high-pressure steam and/or by means of preheated heat-transfer oil.

8. A process according to any one of the preceding claims, characterized in that the bottom of the reactor is heated electrically and/or by means of high-pressure steam and/or by means of preheated heat-transfer oil.

9. A process according to any one of the preceding claims, characterized in that the residue fraction pretreated with methanol is reacted in the methanolysis reactor with further feeding in of methanol.

10. A process according to any one of the preceding claims, characterized in that the residue fraction is worked up in a reaction distillation column.

11. A process according to any one of the preceding claims, characterized in that the reactor bottoms are not subjected to a circulation procedure.

12. A process according to any one of the preceding claims, characterized in that part of the reactor bottoms is bled off and fed to a process for catalyst recovery.

13. A process according to any one of the preceding claims, characterized in that the methanolysis is carried out in a single stage.

14. A process according to any one of the preceding claims, characterized in that useful materials obtainable therein are recirculated to the process.

## Revendications

1. Procédé de traitement d'une fraction résidu par méthanolyse, dans lequel la fraction résidu provient de la distillation d'ester brut du processus de fabrication de DMT,
caractérisé en ce qu'
on ajoute à la fraction résidu du méthanol liquide et/ou sous forme de vapeur, en ce que le mélange possède avant l'entrée dans le réacteur de méthanolyse une température comprise entre 230 et 265°C et en ce que la température dans le bac de décantation du réacteur se situe également entre 230 et 265°C.

2. Procédé selon la revendication 1.
caractérisé en ce que
le mélange possède avant l'entrée dans le réacteur de méthanolyse une température de 245 à 255°C.

3. Procédé selon la revendication 1 ou 2,
caractérisé en ce que
la température dans le bac de décantation du réacteur s'élève à 245 à 255°C.

4. Procédé selon l'une des revendications précédentes,
caractérisé en ce qu'
on procède à la méthanolyse à une pression absolue de 1 à 40 bars.

5. Procédé selon la revendication 4,
caractérisé en ce qu'
on procède à la méthanolyse à une pression absolue de 1 à 3 bars.

6. Procédé selon l'une des revendications précédentes,
caractérisé en ce qu'
on conduit la fraction résidu mélangée de méthanol avant l'introduction dans le réacteur de méthanolyse, dans un échangeur de chaleur.

7. Procédé selon l'une des revendications précédentes,
caractérisé en ce qu'
on fait fonctionner l'échangeur par un moyen électrique et/ou avec une vapeur à haute tension et/ou au moyen d'une huile caloporteuse préchauffée.

8. Procédé selon l'une des revendications précédentes,
caractérisé en ce qu'
on chauffe le bassin de décantation du réacteur par un moyen électrique et/ou avec une vapeur à haute tension et/ou au moyen d'une huile caloporteuse préchauffée.

9. Procédé selon l'une des revendications précédentes,
caractérisé en ce qu'
on fait réagir la fraction résidu prétraitée avec du méthanol, dans le réacteur de méthanolyse, en introduisant encore du méthanol.

10. Procédé selon l'une des revendications précédentes,
caractérisé en ce qu'
on traite la fraction résidu dans une colonne de distillation de la réaction.

11. Procédé selon l'une des revendications précédentes,
caractérisé en ce qu'
on ne soumet le bassin de décantation du réacteur à aucune opération cyclique.

12. Procédé selon l'une des revendications précédentes,
caractérisé en ce qu'
- on évacue une partie du bassin de décantation du réacteur et
- on l'introduit dans un procédé de récupération de catalyseur.

13. Procédé selon l'une des revendications précédentes,
caractérisé en ce qu'
on effectue la méthanolyse en une étape.

14. Procédé selon l'une des revendications précédentes,
caractérisé en ce qu'
on réintroduit dans le processus les matières utiles qu'on y obtient.
